# EUROPEAN PATENT SPECIFICATION

(11) **EP 1 415 639 B1**
(45) Date of publication and mention of the grant of the patent: **27.04.2011**
(21) Application number: 02760584.9
(22) Date of filing: 07.08.2002
(51) Int. Cl.: A61K 8/19, A61Q 1/02, A61Q 1/06

(54) **COMETICS AND MAKEUP METHOD**
KOSMETIKA UND MAKEUP-METHODE
COSMETIQUES ET PROCEDE DE MAQUILLAGE

(30) Priority: 10.08.2001 JP 2001243364; 23.08.2001 JP 2001252457; 23.08.2001 JP 2001252459; 07.05.2002 JP 2002131197; 07.05.2002 JP 2002131198
(43) Date of publication of application: 06.05.2004
(73) Proprietor: Kao Corporation, Chuo-ku Tokyo 103-8210 (JP)
(72) Inventor: KURODA, Akihiro, Odawara-shi, Kanagawa 250-0865 (JP); EGAWA, Yuichiro, Odawara-shi, Kanagawa 250-0055 (JP); SANO, Shoko, Nakano-ku, Tokyo 165-0026 (JP); TOYODA, Takamasa, Odawara-shi, Kanagawa 250-0001 (JP); NIIKUNI, Junko, Odawara-shi, Kanagawa 250-0002 (JP)
(74) Representative: Muschke, Markus Artur Heinz
(86) International application number: PCT/JP2002/008105
(87) International publication number: WO 2003/015723

(56) References cited:
- EP-A- 0 056 094
- WO-A1-00/27345
- WO-A1-00/72808
- JP-A- 9 263 528
- JP-A- 9 315 936
- JP-A- 11 158 036
- JP-A- 2001 114 623
- US-A- 4 839 163
- DATABASE WPI Week 199701 Derwent Publications Ltd., London, GB; AN 1997-007435 XP002377518 & JP 08 277208 A (SUMITOMO CHEM CO LTD.) 22 October 1996 (1996-10-22)

## Description

### TECHNICAL FIELD

The present invention relates to cosmetics that have superior fine wrinkle and skin roughness covering effects as well as skin spots and darkness obscuring effects, exhibit little color darkening over time, allow the obtaining of a cosmetic coating film having a texture close to skin in optical deepness, regulate photographic whitening, and have a superior touch and coloring. In addition, the present invention relates to a makeup method that exhibits the effect of improving cosmetic effects such as improving the coloring of foundation and lipstick.

### BACKGROUND ART

The blending of fine powders of precious stones into cosmetics has heretofore been proposed in an attempt to use inorganic substances having a crystalline structure and transparency in cosmetics.

For example, an ornamental cosmetic composition includes a pure fine powder of a precious stone and a carrier material is disclosed in Japanese PCT Laid-Open Publication No. S63-501362. However, according to this publication, emeralds, which are transparent, turquoise and malachite, which are opaque, and amorphous opal are described to be of the same rank as precious stones, and no attention is paid to effectiveness of specific precious stones with respect to the optical characteristics of cosmetics. Namely, the invention described in this publication attempts to obtain added value of a product by utilizing the ornamental nature of the precious stone itself, while there is no description in this publication that precious stones having a crystalline structure and transparency such as emeralds can be advantageously blended into cosmetics. In other words, in the invention described in this publication, there is consideration whatsoever has been made from the viewpoint of neither the optical properties of precious stones, nor the optical characteristics of the skin after the application of cosmetics to the skin, and particularly the effect of cosmetics of covering fine wrinkles and skin roughness as well as the effect of obscuring skin spots and darkness.

In addition, although Japanese Patent Application Laid-Open Publication No. H10-120520 discloses cosmetics that have been treated by immersing minerals therein, the invention described in the publication also treats transparent quartz and opaque turquoise in the same manner, and the effects of the cosmetics subjected to immersion treatment described in the publication relates to a sense such as mild touch to the skin when cosmetics is treated by immersing, while there is no description whatsoever regarding the optical characteristics of such cosmetics. In addition, numerous technologies have been disclosed in the past regarding diamond powder; for example, Japanese Patent Application Laid-Open Publication No. H6-157263 describes a method of utilizing diamond powder and diamond coated powder as a powder that cuts off infrared rays.

To sum up the descriptions of the aforementioned publications, it has not yet been found that a powder obtained by crushing an inorganic substance having a crystalline structure and transparency in particular possesses superior optical characteristics in the range of visible light.

On the other hand, organopolysiloxane elastomer spherical powders have been frequently used in recent years as materials for exhibiting effects of covering fine wrinkles and roughness in the skin or for changing the texture. However, although organopolysiloxane elastomer spherical powders are highly effective, they have a unique feel, so that the products in which they are blended will resemble each other in touch, and in the case where the material is used universally, there is the problem that it is difficult to differentiate products in terms of their touch. In addition, there are limitations on the amount of the spherical powder that can be blended depending on the preparation. Accordingly, a material that is an inorganic powder and effective is being sought.

In addition, oily solid cosmetics as exemplified by lipstick, and powdered solid cosmetics as exemplified by powder foundation have conventionally used additives such as barium sulfate or covering agents such as pigment-grade titanium oxide to prevent wrinkles on the lips, cheeks, forehead and tail of the eyes and the like from being conspicuous. It is intended to cause sites where the film thickness has become thick due to cosmetics that have entered wrinkles or other grooves to look like other sites where film thickness is thin by scattering light by the additive, or it is intended to cover wrinkles themselves with a covering agent. However, since these materials have considerable effects on the color and texture of the products, in actuality, accommodations are made within the overall balance.

US 4,839,163 describes a facial cosmetic liquid containing a colouring phase containing crystalline mineral silica and colouring material; a major water phase containing water, an emulsifying component and a thickening component; and a minor oil phase containing emolient oils and an emulsifying component.

EP 0 057 266 A2 discloses a cosmetic containing crushed inorganic particles of a particle size in the range of 10 to 50 micrometer selected from pumice powder, quartz flour, corundum, metal carbides, metals and so forth.

WO 00/72808 A1 discloses a cosmetic composition comprising a fluorescent-effective amount of at least one fluorescent mineral powder, in combination with cosmetically acceptable vehicle, wherein the powder, when the composition is applied, does not substantially alter the colour of the skin. The compositions may be used as colour cosmetics and skin treatment products, to replenish the skin's natural fluorescent glow.

WO 87/01278 discloses a cosmetic composition, in particular for aesthetic purposes, containing a liquid, high-viscosity or solid skin-compatible carrier material and pure powder of precious stone mixtures.

### DISCLOSURE OF THE INVENTION

The inventors of the present invention have conducted extensive studies on solutions for the above problems, and found that a powder of a specified grain size obtained by crushing an inorganic substance having a crystalline structure and transparency as represented by some precious stones such as crystal, scatters incident light in multiple directions to give fine wrinkle and skin roughness covering effects as well as skin spots and darkness obscuring effects, and forms light scattering layers in a stepwise manner in the direction of thickness of a cosmetic coating film blended with the powder, to scatter light in a stepwise manner, so that a cosmetic coating film having a texture close to the skin in optical deepness can be obtained.

In addition, it has also been found that the powder, when used in combination with silicone elastomer, can more effectively make fine wrinkles and skin roughness less conspicuous, has a superior touch, and makes color darkening less conspicuous.

Moreover, it has been confirmed that the cosmetic coating film scatters the flash from a camera strobe unit in a direction different from the direction of regular reflection, so that whitening when a picture is taken is suppressed, and it is also capable of presenting an optically attractive expression while preventing wrinkles from being conspicuous during photography in the presence of intense light (such as during television filming), and it has a superior touch as well. Moreover, it has been found that when blended in a powder foundation and so forth, the powder has the effect of making the color darkening over time to appear at a low level, and when the powder is blended in oily cosmetics such as lipstick, the cosmetics exhibit superior coloring.

And in the case where this technology is introduced in cosmetic undercoating materials and finishing cosmetic materials, it has been found that they have the effect of improving cosmetic effects such as improving the coloring of foundation and lipstick used before and after, thereby leading to completion of the present invention.

That is, the present invention relates to a cosmetic as defined in claim 1.

The above cosmetic is one in which the inorganic substance having a crystalline structure and transparency includes sapphire.

The above cosmetic is one that additionally contains a spherical silicone elastomer spherical powder having a primary grain size in the range of 1 to 50 micrometers.

The following cosmetics are disclosed as other preferable modes of the present invention.

The above cosmetic, wherein the size (major axis) of the inorganic substance having a crystalline structure and transparency before crushing is within the range of 0.1 millimeter to 100 centimeters.

The above cosmetic, wherein the shape of the powder obtained by crushing an inorganic substance having a crystalline structure and transparency is irregular or spherical.

The above cosmetic, wherein the inorganic powder is obtained by crushing an inorganic substance having a crystalline structure and transparency in a ball mill or jet mill followed by classification.

The above cosmetic, wherein the inorganic powder obtained by crushing an inorganic substance having a crystalline structure and transparency is further subjected to coating treatment with one kind or more of a surface treatment agent selected from N-acylated lysine, alkylsilane and silicone.

The above cosmetic, wherein the silicone elastomer is blended in one or more kinds of states selected from being pulverized, being mixed by crushing or kneading with an oily agent, and being dispersed in an aqueous system.

The above cosmetic, wherein the blending amount of the powder obtained by crushing an inorganic substance having a crystalline structure and transparency in the cosmetics is in the range of 0.1 to 50% by mass.

The above cosmetic, wherein the blending amount of the powder obtained by crushing an inorganic substance having a crystalline structure and transparency in the cosmetic is in the range of 0.1 to 10% by mass.

The above powdered solid cosmetic that additionally contains another coloring agent and is in a solid state.

The above oily solid cosmetic, further containing an oily agent that is a paste or solid at 25°C and a coloring agent, and being in a solid state.

The following makeup methods are disclosed as preferable modes of use of the present invention.

A makeup method including applying the cosmetic onto a skin as a foundation base and using thereon a cosmetic selected from foundation, eye shadow and lipstick.

A makeup method including applying a cosmetic selected from foundation, eye shadow and lipstick onto a skin and using thereon the above cosmetic as a finishing cosmetic.

A makeup method including applying the above cosmetic to a skin to cover fine wrinkles and skin roughness, obscure skin spots and darkness and regulate whitening during photography.

### BRIEF DESCRIPTION OF THE DRAWINGS

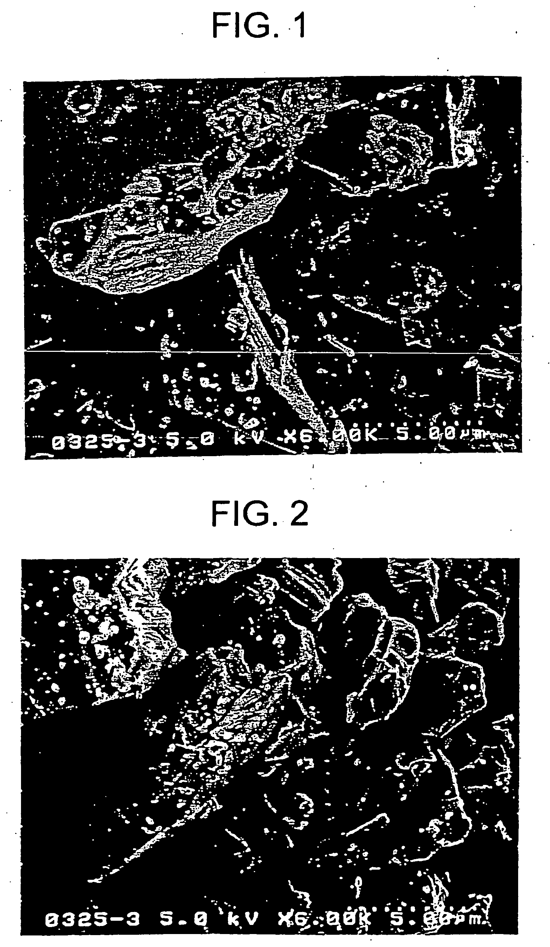

Fig. 1 is an electron micrograph of the shape of quartz powder prior to surface treatment obtained in Production Example 4;
Fig. 2 is an electron micrograph showing the shape of sapphire powder prior to surface treatment obtained in Production Example 5;
Fig. 3 is an electron micrograph showing the shape of ruby powder prior to surface treatment obtained in Production Example 6;
Fig. 4 is a graph showing the reflected light intensities of sapphire powder and of barium sulfate powder prior to surface treatment; and
Fig. 5 is a graph showing the grain size distribution of ground ruby powder (25 micrometer pass) having an average primary grain size of 6.2 micrometers.

### BEST MODE FOR CARRYING OUT THE INVENTION

The inorganic substance having a crystalline structure and transparency used in the present invention refers to a synthetic or natural inorganic sapphire that has a crystalline structure and has optical transparency.

The crystalline structure of the inorganic substance having a crystalline structure as referred to herein may be a single crystal or polycrystalline structure. Examples of crystal forms include an isometric system, a hexagonal system, a trigonal system, a tetragonal system, a monoclinic system, a rhombic system and a triclinic system, and their crystalline structures can be confirmed using X-ray or neutron beam refraction.

Transparency referred to herein indicates that the appearance of an inorganic material at the stage prior to crushing (for example, that of a natural or synthetic rude ore mineral itself) is transparent or semi-transparent. Transparent refers to the state in which light is transmitted and another object can be seen, while semi-transparent refers to the state in which another object is difficult to see even though light is transmitted. Preferably, the material has transparency to the extent that letters and so forth can be read through the rude ore. Note that even in cases in which transparency is not obtained due to scratches and so forth in the surface of a rude ore, materials in which transparency appears as a result of removing the scratched portion of the surface and so forth by polishing the surface are suitable for materials having transparency as referred to in the present invention. In addition, even in the case where an inorganic material after crushing has an opaque appearance due to scratches and so forth in the surface, it falls under the category of the inorganic substance having transparency as referred to herein as long as the rude ore has transparency prior to crushing.

The inorganic substance having a crystalline structure and transparency used in the present invention is such that the inorganic material at the stage prior to crushing has a refractive index of preferably 1.45 to 3.0, and most preferably 1.5 to 2.95. These refractive indices can be measured by a liquid immersion method, a laser measurement method, an apparent depth method, an immersion method or the like. Since the inorganic material at the stage prior to crushing has a refractive index in this range shows scattering phenomena over a wide range when light is irradiated to an inorganic material after crushing , a cosmetic coating film is obtained that has superior fine wrinkles and skin roughness covering effects and superior skin spot and darkness obscuring effects to light scattering pigments conventionally used .

Specific examples of such substances include garnet, spinel, scapolite, rutile, zircon, vesuvianite, emerald, aquamarine, heliodor, goshenite, morganite, red beryl, crystal, amethyst, citrin, rose quartz, brown quartz, milky quartz, chalcedony, ruby, sapphire, padparadscha, phenakite, topaz, chrysoberyl, alexandrite, enstatite, silimanite, hypersthene, peridot, spodumene, titanite, quartz and synthetic quartz, and those sapphires having a crystalline structure and transparency are selected for use according to the invention.

In particular, since these inorganic substances can be supplied artificially, and since they do not contain lead, arsenic or radioactive substances and so forth so that they have no effects thereof, they have merits that they are capable of imparting preferable qualities in terms of cosmetics and that they are in a stable supply.

Diamonds, which are well known as precious stones having transparent crystals, are too hard to effectively control the grain size by crushing and grinding, so that a powder in a region of superior optical characteristics that is the object of the present invention cannot be obtained. On the other hand, even if the inorganic substance is a precious stone having a crystalline structure and transparency, the one which lacks stability in a humid atmosphere even when subjected to surface treatment as is the case with apatite or calcite, the one which contains elements such as lead that are not suitable as raw material ingredients of cosmetics as is the case with cerussite, and the one which easily changes color due to heat in post-treatment and so forth as is the case with tourmaline (borate silicate salt), are excluded.

The powders of sapphire obtained by crushing inorganic substances having a crystalline structure and transparency used in the present invention are generally obtained by crushing in a broad sense including crushing, grinding or polishing of a synthetic or naturally-occurring inorganic material that has grown and been crystallized to a primary grain size of 100 micrometers or more, while a powder obtained by grain growth directly from clusters without going through an act of crushing and so forth does not fall under the category of the powder used in the present invention due to the presence of lattice defects and to inferior optical characteristics of the powder.

Although there are no particular restrictions on the powder obtained by crushing an inorganic substance having a crystalline structure and transparency used in the present invention so far as it satisfies the aforementioned criteria, synthetic or natural precious stones that are generally referred to as precious stones and have a crystalline structure and transparency are preferable. It is preferable to use synthetic or natural inorganic substances in which the size (major axis) of the inorganic raw material before crushing during mechanical crushing is in the range of from 0.1 millimeter to 100 centimeters. This is because precious stones of less than 0.1 millimeter may have problems of containing large amounts of impurities, exhibiting poor crystallinity, having crystal lattice defects and having inferior optical characteristics of powder, while those of larger than 100 centimeters may have the problem of requiring excessive time and bother until crushing.

Hereinafter, differences between the present invention and the materials used in conventional cosmetics, made of silica and alumina, which are the same elements as those being sought in the present invention will be described. Materials such as silica and alumina have been frequently used in conventional cosmetics. The elementary composition of these materials includes two or more elements selected from the silica, aluminum and oxygen sought in the aforementioned powder of the inorganic substance having a crystalline structure and transparency, and there are some aspects in which the constituent elements coincide therewith. However, the majority of these materials are obtained not by crushing grains, but rather by growing grains, and they are amorphous and have numerous internal lattice defects, so that they are opaque. Not only since these powders are frequently used conventionally, but also since they do not have optical characteristics to a degree that enables them to exhibit the effects required by the present invention, powders that are normally used in cosmetics such as silica (including spherical silica), alumina, and zirconia are not included in the present invention. In addition, although many of the titanium oxide and zinc oxide normally used in cosmetics have a crystalline structure, they are excluded since the appearance of the powder is opaque so that the optical effect required in the present invention cannot be obtained. Thus, in the case where the titanium oxide (rutile) that can be used in the present invention is referred to, it indicates a crushed substance of a single crystal. Also, for glasses, crystallized glass having crystallinity is applicable to the present invention while typical amorphous glass is not applicable.

The powder of sapphire used in the present invention that is obtained by crushing an inorganic substance having crystallinity and transparency has an average primary grain size in the range of 5 micrometers to 15 micrometers, and is classified so that the number of grains having a primary grain size of 25 micrometers or more is less than 15% and as few as possible. In addition, the grain size distribution is preferably such that the number of grains having a primary grain size of 1 micrometer or less is not greater than 20%. If the average primary grain size is less than 3 micrometers, the amount of energy required for crushing becomes large, which leads to increased costs and in addition, to loss of the optical characteristics targeted by the present invention, so that the effects of the present invention cannot be obtained. In addition, if the average primary grain size is greater than 20 micrometers, there are cases where the feel of cosmetics when the powder is blended therein becomes coarse. Since the average primary grain size in the range of 5 to 15 micrometers is the range at which optical effects are expressed most strongly, it is advantageous to set crushing conditions so that the average primary grain size is in this range as much as possible, with the result that the desired optical effects can be obtained easily even in a preparation.

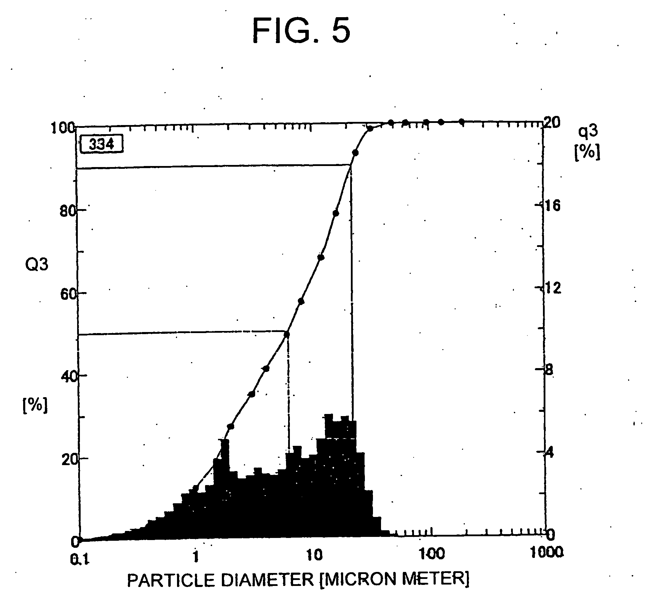

The average primary grain size referred to here indicates t is measured in an ethanol dispersion solvent using, for example, an ordinary laser grain size distribution meter (such as the Model PRO-7000S laser grain size distribution meter manufactured by Seishin Enterprise Co., Ltd.). For example, the grain size distribution of ruby powder (25 micrometer pass preparation) having an average primary grain size of 6.2 micrometers is shown in Fig. 5. In addition, the average primary grain size can also be measured by image processing of electron micrographs.

Production methods of the powder obtained by crushing an inorganic substance having crystallinity and transparency that is used in the present invention include a method in which single crystals or polycrystalline substances of the aforementioned inorganic materials are pulverized using a dry or wet crusher or grinder such as a jet mill, a hammer mill, a ball mill, a vibrating ball mill, a cutter mill, a bead mill or Micros manufactured by Nara Machinery Co., Ltd.), and methods that use combinations of the above. Particularly, in the present invention, pulverizing using a ball mill (including a vibrating ball mill) or a jet mill makes it easy to obtain a powder of the target average primary grain size. In addition, after the crushing, the optical characteristics of the product can be improved by classifying using a classifying device such as a turbo screener, a vacuum sieve, a vibrating sieve or an ultrasonic sieve.

In the present invention, there are no particular restrictions on the shape of the grains; however, if the shape of the grains is irregular or spherical, the optical diffusion characteristics are improved, so that these shapes are preferable. Moreover, when the grain size distribution is viewed in terms of volumetric average grain size, the grain size distribution may be either sharp or broad. If the grain size distribution is sharp, there is less of a mat sense and lustrous cosmetics tend to be obtained. If the grain size distribution is broad, light diffusion and scattering effects become stronger, and this results in stronger wrinkle and fine wrinkle covering effects; however, the mat sense tends to become relatively strong.

Incidentally, in the case where a cosmetic contains a large amount of powder of 25 micrometers or larger, there are cases when the feel of the cosmetic becomes coarse even though the average primary grain size was in the preferable range. Consequently, it is preferable to restrict the total amount of grains having a grain size of 25 micrometers or larger by, for example, passing through a 25 micrometers mesh. In addition, in the present invention, these powders may be preliminarily subjected to an impurity removal treatment using an acid solution such as a hydrochloric acid solution or an alkali solution such as a sodium hydroxide solution in order to remove impurities.

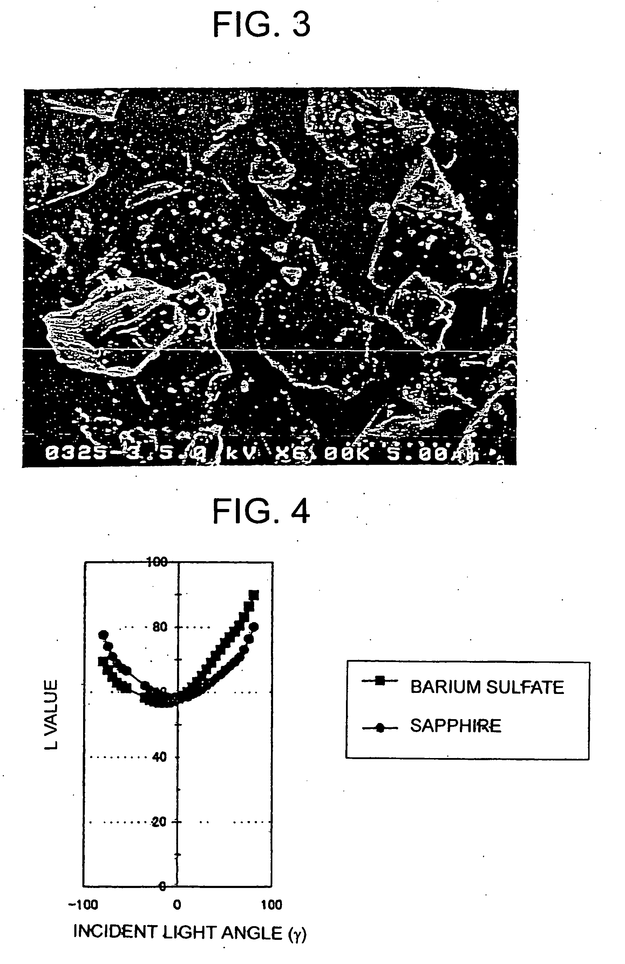

The reflected light intensities of powder obtained by crushing sapphire with a ball mill and of a barium sulfate powder frequently used as a wrinkle covering pigment in ordinary lipstick and so forth were evaluated using the following device as examples of powder obtained by crushing the inorganic substance having crystallinity and transparency used in the present invention The results are shown in Fig. 4. In addition, based on the results of Fig. 4, it can be seen that in contrast to the conventional light-scattering pigment, barium sulfate, that reflects light non-uniformly (lateral symmetry is not observed), the powder obtained by crushing sapphire is laterally symmetrical and scatters light uniformly, and therefore is preferable.

### [Reflected Light Intensity Measurement Device and Method]

Device manufacturer: Murakami Color Research Laboratory
Device model name: Model GCMS-4 Gonio Color Measuring System
Incident light angle: 45 degrees

The powder obtained in this manner is then preferably subjected to coating treatment with a water repellent or hydrophilic surface treatment agent. Examples of the water repellent or hydrophilic coating treatment include a fluorine compound treatment, a silicone resin treatment, a silicone treatment, a pendant treatment, a silane coupling agent treatment, a titanium coupling agent treatment, an oily agent treatment, an N-acylated lysine treatment, a polyacrylic acid treatment, a metallic soap treatment, an amino acid treatment, a plasma treatment and a mechanochemical treatment, and these may be performed alone or in a combination of a plurality of treatments. However, an N-acylated lysine treatment using, for example, N^{ε}-lauroyl-L-lysine (Amihope LL, Ajinomoto), and alkylsilane treatment using, for example, octyltrialkoxysilane are particularly preferable. The amount of treatment in this case is preferably in the range of 0.5 to 15% by mass with respect to the weight of the powder.

In addition, the silicone treatment is also preferable, examples of which include treatment with methyl hydrogen polysiloxane (e.g., KF99P manufactured by Shin-Etsu Chemical), dimethyl group-containing methyl hydrogen polysiloxane (e.g., KF9901, HRS-2, manufactured by Shin-Etsu Chemical) or cyclic methyl hydrogen polysiloxane (e.g., KF9902, manufactured by Shin-Etsu Chemical) and its baking treatment. Moreover, it is also preferable to perform surface treatment with an inorganic oxide such as silica, alumina or zirconia prior to water repellent surface treatment. In addition, examples of coating treatment using a hydrophilic surface treatment agent include an inorganic oxide treatment, and a surface treatment using a water-soluble or water-swelling thickener such as agar, alginic acid and its salts, hyaluronic acid and its salts, deoxyribonucleic acid and its salts, pullulan, guar gum, gellan gum, polyacrylic acid and its salts. The amount of surface treatment other than the alkylsilane treatment is, for example, preferably 0.1 to 100 parts by mass, and more preferably 0.3 to 30 parts by mass, with respect to 100 parts by mass of the powder.

The powder obtained by crushing an inorganic substance having a crystalline structure and transparency used in the present invention is preferably blended at 0.1 to 98% by mass, and more preferably 0.1 to 50% by mass, in cosmetics. In the case of oily cosmetics in particular, the powder is preferably blended in the range of 0.1 to 10% by mass. If the blending amount is less than 0.1 % by mass, there are cases in which the optical effects of the present invention are not obtained. On the other hand, there are cases in which effects are not obtained that are comparable to the blending amount, even if the blending amount exceeds 50% by mass.

Many of the powders obtained by crushing an inorganic substance having a crystalline structure and transparency used in the present invention have a high hardness, and there are cases in which wear of the mixing device and so forth are caused when mixing is carried out with an inorganic powder having a crystalline structure and transparency alone, so that it is preferable to carry out mixing with a component that can serve as a lubricant such as resin powder or talc. Moreover, since the powder used in the present invention frequently has a larger specific gravity than each of the pigments indicated below, caution is required so that incomplete mixing does not occur during mixing. For example, in the production of a powder foundation, a method can be employed in which the powder of the present invention is added during the latter half of the mixing step so that mixing can be carried out while reducing the burden on the device. In addition, in order to prevent wear of the device, it is also preferable to use a ceramic-coated mixing device.

In the present invention, a silicon elastomer is blended together with the aforementioned inorganic powder having a crystalline structure and transparency. The cosmetic of the present invention comprises spherical silicone elastomer powder having a primary grain size in the range of 1 to 50 micrometers. Although these are known to have optical effects that obscure wrinkles, those effects were not necessarily satisfactory in the case of silicone elastomer alone.

A spherical powder having a primary grain size in the range of 1 to 50 micrometers is used as the silicon elastomer, while a spherical powder having a primary grain size of 1 to 15 micrometers is used more preferably. Although examples of silicon elastomers include members of the Torayfil E series of Toray-Dow Corning and members of the KSG series of Shin-Etsu Chemical, spherical powder of the Torayfil E series (such as Torayfil E-505C, Torayfil E-506C, Torayfil E-507 and Torayfil E-508) are preferable since they are highly effective in obscuring wrinkles. Silicon elastomers are obtained by reacting methyl hydrogen polysiloxane and silicone modified with olefin at both ends in the presence of a platinum-based catalyst.

These powders are rich in rubber elasticity unless subjected to modification treatment, and when observed with an electron microscope, they can be observed to be in a state in which the spherical primary grains are agglomerated in the form of high-order agglomerate in a shape like a grape cluster. These agglomerate grains are extremely large, and many reach a size of several hundred micrometers to several millimeters. Since this powder is rich in elasticity, the powder cannot be crushed properly in dry impact crushers used for ordinary cosmetics (such as a pin mill or a hammer mill), with the force of impact being absorbed. The grains are characterized that since the grains themselves are very soft, attempts to classify them with a mesh sieve result in generation of static electricity, which causes the grains to fly around and at the same time join with each other, so that fine grains cannot be obtained.. Therefore, it is preferable to blend a powder on which crushing has been performed using a dry crusher such as a cutter mill, a turbo mill or an impeller mill that has a shearing mechanism, preferably has a classifying mechanism and also preferably uses a high-speed rotating type of crusher, or a powder on which crushing has been performed using a roller mill or a wet medium type of crusher such as a bead mill, a sand mill or Micros (manufactured by Nara Machinery Co., Ltd.).

In the case of using a dry crusher, crushing is preferably carried out together with a lubricant such as a plate-like powder (talc, mica, sericite, kaolin, boron nitride, plate-like barium sulfate, plate-like silicic anhydride, plate-like titanium oxide, N-lauroyl-L-lysine, metallic soap pigment, mica titanium, etc.), silicic anhydride, silicone powder, e.g., polymethylsilsesquioxane, and spherical silica beads.

In addition, in the case of using a wet medium-crusher or a roller mill, it is preferable to blend a dispersion of a silicone elastomer obtained by crushing or kneading together with an oily agent or water. Namely, blending the silicone elastomer in one or more states selected from that which has been pulverized, that which has been mixed with an oily agent by crushing or kneading and that which has been dispersed in an aqueous system is preferable in terms of the feel of the cosmetics and the object of the present invention, and in particular it is preferable to blend that which has been put into one of the aforementioned states in advance in the cosmetics.

There are no particular problems in using any mixing oily agent as far as it is one of the oily agents listed below that is generally used in cosmetics; however, silicone oils, ester oils and hydrocarbon oils are particularly preferable. In addition, in the case of using an aqueous dispersion, it is preferable to use this in combination with a surfactant during crushing. The crushed powder and so forth obtained in this manner frequently adopt the form of generally an agglomerate in which several primary grains agglomerate.

The silicone elastomer used in the present invention may or may not undergo the various types of surface treatment previously described. In the case of performing surface treatment, a silicone-based treatment agent having superior compatibility such as a silicone resin is preferable.

The blending amount of a silicone elastomer in the cosmetics in the present invention is preferably in the range of 0.1% by mass to 30% by mass, and particularly preferably in the range of 0.5% by mass to 20% by mass. If the blending amount is less than 0.1% by mass, there are cases in which the optical effects of the present invention may be diminished.

In the present invention, it is also possible to blend a powder coated with a silicone elastomer, for example, by making the silicone elastomer an aqueous dispersion, mixing and drying the dispersion with another powder to coat the elastomer around the powder.

In the present invention, in addition to the aforementioned powder obtained by crushing an inorganic substance having a crystalline structure and transparency, those components ordinarily blended into cosmetics such as various types of pigments, ultraviolet absorbents, oily agents, fluorine compounds, resins, thickeners, antiseptics, fragrances, moisturizers, salts, solvents, antioxidants, chelating agents, neutralizers, pH adjusters, insect repellents and physiologically active components can also be used.

Examples of the oily agents used in the present invention include avocado oil, linseed oil, almond oil, insect wax, eno oil, olive oil, cacao butter, kapok wax, Japanese torreya oil, carnauba wax, liver oil, candelilla wax, apricot kernel oil, hardened oil, wheat germ oil, sesame oil, rice germ oil, rice bran oil, sugarcane wax, *Camellia sasanqua* oil, safflower oil, shea butter, China wood (*Paulownia fortunei Hemsley*) oil, cinnamon oil, jojoba wax, shellac wax; turtle oil_{;} soybean oil, tea seed oil, camellia oil, evening primrose oil, corn oil, rape seed oil, Japan tung oil, rice bran wax, germ oil, persic oil, palm oil, palm kernel oil, castor oil, hardened castor oil, castor oil fatty acid methyl ester, sunflower oil, grape oil, bayberry wax, jojoba oil, macadamia nut oil, bees wax, cotton seed oil, cotton wax, Japan wax, Japan wax kernel oil, montan wax, coconut oil, hardened coconut oil, tri(coconut oil fatty acid) glyceride, peanut oil, lanolin, liquid lanolin, reduced lanolin, lanolin alcohol, hard lanolin, lanolin acetate, isopropyl lanolin fatty acid, hexyl laurate, POE lanolin alcohol ether, POE lanolin alcohol acetate, polyethylene glycol lanolin fatty acid, POE hydrogenated lanolin alcohol' ether, and egg yolk oil; hydrocarbon oils such as ozocerite, squalane, squalene, ceresin, paraffin, paraffin wax, liquid paraffin, pristane, polyisobutylene, micro-crystalline wax and vaseline; higher fatty acids such as lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid, and 12-hydroxystearic acid; higher alcohols such as lauryl alcohol, myristyl alcohol, palmityl alcohol, stearyl alcohol, behenyl alcohol, hexadecyl alcohol, oleyl alcohol, isostearyl alcohol, hexyldodecanol, octyldodecanol, cetostearyl alcohol, 2-decyltetradecynol, cholesterol, phytosterol, POE cholesterol ether, glycerin monostearyl ether (batyl alcohol) and monooleyl glyceryl ether (selachyl alcohol); ester oils such as diisobutyl adipate, 2-hexyl decyl adipate, di-2-heptyl undecyl adipate, n-alkyl glycol monoisostearate, isocetyl isostearate, trimethylolpropane triisostearate, ethylene glycol di-2-ethylhexanoate, cetyl 2-ethylhexanoate, trimethylolpropane tri-2-ethylhexanoate, pentaerythritol tetra-2-ethylhexanoate, cetyl octanoate, octyldodecyl gum ester, oleyl oleate, octyldodecyl oleate, decyl oleate, isononyl isononanoate, neopentyl glycol dicaprate, triethyl citrate, 2-ethylhexyl succinate, amyl acetate, ethyl acetate, butyl acetate, isocetyl stearate, butyl stearate, diisopropyl sebacate, di-2-ethylhexyl sebacate, cetyl lactate, myristyl lactate, isopropyl palmitate, 2-ethylhexyl palmitate, 2-hexyldecyl palmitate, 2-heptylundecyl palmitate, cholesteryl 12-hydroxystearate, dipentaerythritol fatty acid esters, isopropyl myristate, octyldodecyl myristate, 2-hexyldecyl myristate, myristyl myristate, hexyldecyl dimethyloctanoate, ethyl laurate, hexyl laurate, N-lauroyl-L-glutamic acid-2-octyl dodecyl ester, and diisostearyl malate; glyceride oils such as acetoglyceryl, glyceryl triisooctanoate, glyceryl triisostearate, glyceryl triisopalmitate, glyceryl monostearate, glyceryl di-2-heptylundecanoate, glyceryl trimyristate and diglyceryl myristate isostearate.

In the present invention, oily solid cosmetics such as lipsticks obtained by blending, among the aforementioned components, the powder obtained by crushing an inorganic substance having a crystalline structure and transparency, an oily agent that is a paste or solid at 25°C and in addition a coloring material are used preferably. The oily agent that is a paste or solid at 25°C as referred to herein includes oily agents and resins conventionally used in cosmetics, as well as resins and thickeners that become a paste or solid at 25°C by dissolving in another oily agent. Typical examples of these include carnauba wax, candelilla wax, polyethylene wax, rice bran wax, jojoba wax, shellac, lanolin, bees wax, white beeswax, ozocerite, ceresin, paraffin, microcrystalline wax, ethylene propylene copolymer, vaseline, polybutene, lauric acid, myristic acid, palmitic acid, stearic acid, behenic acid, oleic acid, isostearic acid, 12-hydroxystearic acid, dextrin palmitate, undecylenic acid, cetanol, behenyl alcohol, batyl alcohol, chimyl alcohol, selachyl alcohol, isostearyl glyceryl ether, isostearic acid hardened castor oil, caprylic/capric/myristic/stearic triglyceride, glyceryl triundecylate, pentaerythritol tetramyristate, hydrogenated milk fats, cholesteryl lanolin fatty acid, cholesteryl hydroxystearate, cholestery oleate, dihydrocholesteryl oleate, phytosteryl oleate, phytosteryl isostearate, di(cholesteryl · behenyl · octyl · dodecyl) N-lauroyl-L-glutamate, di(phytosteryl · behenyl · octyl · dodecyl) N-lauroyl-L-glutamate, cholesteryl macadamia nut oil fatty acid, phytosteryl macadamia nut oil fatty acid, di(isostearyl · phytosteryl) dimer dilinoleate, dimer dilinoleyl dimer dilinoleate, polyethylene glycol, polyglycerin, palm oil, coconut oil, hardened oil, horse oil, shea butter, and egg yolk oil as well as resins such as polypropylene, polytetrafluoroethylene, acryl silicone, acrylic acid polymer and salts thereof, acrylic acid methacrylic acid copolymer and salts thereof, and polyvinylpyrrolidone.

The blending amount of an oily agent used in the present invention that is a paste or solid at 25°C is preferably 0.5 to 60% by mass with respect to the total amount of oily solid cosmetic.

In addition, in the present invention, various types of oily agents, for example, silicone oil and fluorine-based oily agents normally used in cosmetics can also be blended in addition to the aforementioned oily agents. Examples of these include silicone compounds such as dimethylpolysiloxane, methylphenylpolysiloxane, acrylic-modified organopolysiloxane, fluorine-modified organopolysiloxane, polyether-modified organopolysiloxane, amino-modified organopolysiloxane, alcoholic hydroxyl group-containing organopolysiloxane, glyceryl-modified organopolysiloxane, polyglyceryl-modified organopolysiloxane that may have a branched siloxane chain, sugar-modified organopolysiloxane, alkyl-modified organopolysiloxane modified with alkyl other than methyl or phenyl, amodimethicone, perfluoroalkylated dimethiconol, perfluoroalkyl-polyoxyalkylene-co-modified silicone, highly polymerized silicone, cyclic volatile silicone and methyltrimethicone, fluorocarbons such as perfluoropolyether, pitch fluoride, perfluorodecaline and perfluorooctane, and fluorine-based oily agents such as fluoroalcohols and perfluoroalkyl alkyl ethers.

In addition to the aforementioned oily solid cosmetics, powder foundations, cheek colors, rouge and other powdered solid cosmetics are suitable as cosmetics of the present invention. Examples of the coloring materials used in these oily solid cosmetics and powdered solid cosmetics include various pigments normally used in cosmetics, with organic coloring matter and pearl pigments being particularly preferable. Examples of the pigments include inorganic powders such as pigment-grade titanium oxide, zirconium oxide, pigment-grade zinc oxide, cerium oxide, magnesium oxide, barium sulfate, calcium sulfate, magnesium sulfate, calcium carbonate, magnesium carbonate, talc, mica, kaolin, sericite, white mica, synthetic mica, gold mica, crimson mica, black mica, lithia mica, silicic acid, silicic acid anhydride, aluminum silicate, magnesium silicate, aluminum magnesium silicate, calcium silicate, barium silicate, strontium silicate, tungstic acid metal salt, hydroxyapatite, vermiculite, Higilite (trademark), bentonite, montmorillonite, hectorite, zeolite, ceramics powder, calcium secondary phosphate, alumina, aluminum hydroxide, boron nitride, silica, silica-treated fine grain titanium oxide, fine grain titanium oxide and fine grain zinc oxide, fine grain cerium oxide and the like; organic powders such as polyamide powder, polyester powder, polyethylene powder, polypropylene powder, polystyrene powder, polyurethane powder, benzoguanamine powder, polymethylbenzoguanamine powder, polytetrafluoroethylene powder, polymethyl methacrylate powder, cellulose, silk powder, nylon powders such as nylon 12 powder and nylon 6 powder, polymethyl silsesquioxane, styrene acrylic acid copolymer, divinylbenzene styrene copolymer, vinyl resin, urea resin, phenol resin, fluororesin, acrylic resin, melamine resin, epoxy resin, polycarbonate resin, crystallite fiber powder, starch powder and lauroyl lysine and the like; surface active agent metal salt powders (metallic soaps) such as zinc stearate, aluminum stearate, calcium stearate, magnesium stearate, zinc myristate, magnesium myristate, zinc cetyl phosphate, calcium cetyl phosphate and zinc cetyl phosphate; colored pigments such as inorganic red pigments, e.g., iron oxide, iron hydroxide, and iron titanate, inorganic brown pigments, e.g., γ-iron oxide, inorganic yellow pigments, e.g., yellow iron oxide and loess, inorganic black pigments, e.g., black iron oxide and carbon black, inorganic purple pigments, e.g., manganese violet and cobalt violet, inorganic green pigments, e.g., chromium hydroxide, chromium oxide, cobalt oxide, and cobalt titanate, inorganic blue pigments, e.g., Prussian blue and ultramarine blue, pearl pigments such as titanated mica and bismuth oxychloride, laked tar-based pigments, laked natural pigments, and synthetic resin powders in which these powders are compounded and the like; tar pigments such as Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206. and Orange No. 207; natural pigments such as powders selected from carminic acid, laccaic acid, carthamin, brazilin and crocin. These pigments may or may not undergo various types of surface treatment similar to that previously described.

The blending amount of coloring material used in the present invention is preferably 0.3 to15% by mass with respect to the total amount of oily solid cosmetic in terms of the total amount of blended coloring material, and in the case of powdered solid cosmetics, the blending amount is preferably 60 to 90% by mass with respect to its total amount. Note that although the aforementioned inorganic powder having a crystalline structure and transparency used in the present invention is also a type of coloring material in the broad sense, it is treated as a different material here. Thus, the blending amount of inorganic powder having crystallinity and transparency is not included in the blending amount of the aforementioned coloring material.

Although examples of surfactants used in the present invention include anionic, cationic, non-ionic and amphoteric surfactants, there are no particular restrictions on the surfactant used, and any surfactant can be used as far as it is used in ordinary cosmetics. Specific examples of the surfactants are as described hereinbelow. Anionic surfactants include fatty acid soaps such as sodium stearate and triethanolamine palmitate, alkyl ether carboxylic acids and salts thereof, carboxylic acid salts such as condensate between amino acid and fatty acid, alkyl sulfonic acids, sulfonic acid salts such as alkenesulfonic acid salts, fatty acid ester sulfonic acid salts, fatty acid amide sulfonic acid salts, and alkylsulfonic acid salts and formalin condensates thereof, sulfuric acid ester salts such as alkyl sulfate salts, the secondary higher alcohol sulfate salts, alkyl and aryl ether sulfate salts, sulfate salts of fatty acid esters, sulfate salts of fatty acid alkylolamide and turkey red oil, alkyl phosphate salts, ether phosphate salts, alkyl aryl ether phosphate salts, amide phosphate salts, N-acylamino acid based activators, etc.; cationic surface active agents include amine salts such as alkylamine salts, polyamines and amino alcohol fatty acid derivatives, alkyl quaternary ammonium salts, aromatic quaternary ammonium salts, pyridinium salts and imidazolium salts, etc.; nonionic surfactants such as sorbitan fatty acid esters, glycerin fatty acid esters, polyglycerol fatty acid esters, propylene glycol fatty acid esters, polyethylene glycol fatty acid esters, sucrose fatty acid esters, polyoxyethylene alkyl ethers, polyoxypropylene alkyl ethers, polyoxyethylene alkyl phenyl ethers, polyoxyethylene fatty acid esters, polyoxyethylene sorbitan fatty acid esters, polyoxyethylene sorbitol fatty acid esters, polyoxyethylene glycerin fatty acid esters, polyoxyethylene propylene glycol fatty acid esters, polyoxyethylene castor oil, polyoxyethylene hardened castor oil, polyoxyethylene phytostanol ether, polyoxyethylene phytosterol ether, polyoxyethylene cholestanol ether, polyoxyethylene cholesteryl ether, alkanolamides, sugar ethers such as dodecyl raffinose and alkyl glycosides, sugar amides; and amphoteric surfactants such as betaine, amino carboxylates and imidazoline derivatives. The blending amount of surfactant is preferably 0.1 to 20% by mass, and more preferably 0.5 to 10% by mass, with respect to the total amount of cosmetics. In addition, one type or two or more types of surfactants may be used.

Examples of ultraviolet absorbents include octyl paramethoxycinnamate, 2-hydroxy-4-methoxybenzophenone, 2-hydroxy-4-methoxybenzophenone-5-sulfuric acid, 2,2'-dihydroxy-4-methoxybenzophenone, p-methoxyhydrocinnamic acid diethanolamine salt, para-aminobenzoic acid (hereinafter abbreviated to PABA), ethyl dihydroxypropyl PABA, glyceryl PABA, homomenthyl salicylate, methyl-O-aminobenzoate, 2-ethylhexyl-2-cyano-3,3-diphenyl acrylate, octyl dimethyl PABA, octyl salicylate, 2-pheryl-benzimidazele-5-sulfuric acid, triethanolamine salicylate, 3-(4-methylbenzylidene)camphor, 2,4-dihydroxybenzophenine, 2,2',4,4'-tetrahydroxybenzophenone, 2,2'-dihydroxy-4,4'-dimethoxybenzophenone, 2-hydroxy-4-N-octoxybenzophenone, 4-isopropyldibenzoylmethane, butylmethoxydibenzoylmethane, 2-ethylhexyl 4-(3,4-dimethoxyphenylmethylene)-2,5-dioxo-1-imidazolidine propionate, octyltriazone, dimethoxycinnamyl isooctanoyl glyceride and their polymer derivatives, silane derivatives and the like.

In addition, organic ultraviolet absorbents entrapped within a polymer powder can also be used. The polymer powder may or may not be hollow, and advantageously have an average primary grain size within the range of 0.1 to 50 micrometers; the grain size distribution may be broad or sharp. Examples of polymer types include acrylic resin, methacrylic resin, styrene resin, urethane resin, polyethylene, polypropylene, polyethylene terephthalate, silicone resin, nylon and acrylamide resin. The organic ultraviolet absorbent can have further enhanced ultraviolet protective effects when used in combination with an inorganic ultraviolet scattering agent such as fine grain titanium oxide, fine grain zinc oxide and fine grain cerium oxide.

When using an ultraviolet absorbent in the present invention, there are cases where it is preferable to use the aforementioned powder obtained by crushing an inorganic substance having crystallinity and transparency of the present invention that has further been treated by firing. Those powders that require this treatment in particular are powders having silicon in their skeleton. If quartz powder and so forth is allowed to contact an ultraviolet absorbent without undergoing this treatment, yellow to purple coloring that is believed to be caused by discoloration of the ultraviolet absorbent may occur in some cases. Furthermore, there are also cases in which the coloring of the powder itself becomes more intense due to firing depending on the type of powder used. Firing conditions include a temperature range of 300 to 1000°C, and preferably 500 to 800°C, and a firing time of 0.1 to 24 hours. A gas or electric firing oven can be used for firing. In addition, performing hydrophobic surface treatment on the fired powder makes it possible to further enhance the stability of the powder.

Examples of antimicrobial antiseptics include paraoxybenzoic acid alkyl esters, benzoic acid, sodium benzoate, sorbic acid, potassium sorbate and phenoxyethanol and the like, while examples of antibacterial agents include benzoic acid, salicylic acid, carbolic acid, sorbic acid, paraoxybenzoic acid alkyl esters, parachlorometacresol, hexachlorophene, benzalkonium chloride, chlorohexidine chloride, trichlorocarbanilide, triclosan, photosensitive materials and phenoxyethanol and the like.

Examples of thickeners and resins include silicone compounds such as trimethylsiloxy silicic acid, fluorinated silicone resin and cationized silicone resin, vegetable based polymers such as gum arabic, gum tragacanth, arabinogalactan, locust bean gum (carob gum), guar gum, Karaya gum, carrageenan, pectin, agar, quince seed (quince), starch (rice, corn, potato, wheat), algecolloid, microbe-based polymers such as xanthan gum, dextran, succinoglucan, pullulan and siliconated pullulan, starch-based polymers such as carboxymethyl starch and methyl hydroxypropyl starch, cellulose-based polymers such as methyl cellulose, ethyl cellulose, methyl hydroxypropyl cellulose, carboxymethyl cellulose, hydroxymethyl cellulose, hydroxypropyl cellulose, nitrocellulose, cellulose sodium sulfate, sodium carboxymethyl cellulose, crystalline cellulose and cellulose powder, alginic acid-based polymers such as sodium alginate and propylene glycol alginate, vinyl polymers such as polyvinyl methyl ether and carboxyvinyl polymer, polyoxyethylene-based polymer, polyoxyethylene polyoxypropylene copolymer-based polymer, acrylic-based polymers such as sodium polyacrylate, polyethyl acrylate and polyacrylic acid amide, inorganic-based water-soluble polymers such as polyethyleneimine, cationic polymer, bentonite, aluminum magnesium silicate, raponite, hectorite and silicic acid anhydride, polyethylene glycol, polyvinylpyrrolidone, and the like.

Various types of physiologically active components are preferably blended into the cosmetics according to the present invention. Examples of physiologically active components used in the present invention include substances that impart some type of physiological activity to the skin when applied to the skin. Examples of physiologically active components include whitening components, anti-inflammatory agents, aging preventives, slimming agents, tightening agents, antioxidants (radical capturers), moisturizers, circulation promoters, antibacterial agents, disinfectants, siccatives, cooling agents, warming agents, vitamins, amino acids, wound healing promoters, irritation relaxants, analgesics, cell activators, skin colorants and enzyme components. Among these, natural plant extract components, algae extract components and herbal medicine components are particularly preferable. In the present invention, it is preferable to blend one type or two or more types of these physiologically active components.

Examples of these physiologically active components include ashitaba (*Angelica keiskei*) extract, avocado extract, sweet hydrangea leaf extract, althea extract, arnica extract, aloe extract, apricot extract, apricot kernel extract, *Ginkgo biloba* extract, fennel extract, turmeric extract, oolong tea extract, rose fruit extract, *Echinacea* leaf extract, *Scutellaria* root extract, *Phellodendron* bark extract, Japanese *Coptis* extract, barley extract, *Hypericum* extract, white nettle (*Lamium album*) extract, watercress extract, orange extract, sea water dry matter, sea weed extract, hydrolyzed elastin, hydrolyzed wheat powder, hydrolyzed silk, cassis extract, chamomile extract, carrot extract, *Artemisia capillaris* extract, licorice extract, carcade extract, *Pyracantha fortuneana* fruit extract, kiwi extract, cinchona extract, cucumber extract, guanosine, gardenia extract, *Sasa veitchii* extract, *Sophora flavescens* extract, walnut extract, grapefruit extract, *Clematis* leaf extract, *Chlorella* extract, mulberry root extract, gentian extract, black tea extract, yeast extract, burdock root extract, rice bran fermentation extract, rice germ oil, comfrey extract, collagen, mountain cranberry (*Vaccinium vitis-idaea*) extract, *Ssarum sieboldii* extract, *Bupleurum* root extract, umbical cord extract, salvia extract, *Saponaria* extract, sasa-bamboo extract, *Crataegus* extract, *Zanthoxylum* fruit extract, shiitake mushroom extract, *Rehmannia* root extract, *Lithospermum* root extract, *Perilla frutescens* extract, *Tilia japonica* extract, meadowsweet (*Filipendula ulmaria*) extract, peony extract, *Acorus calamus* root extract, birch bark extract, horsetail extract, ivy extract, *Crataegus monogina* extract, *Sambucus nigra* extract, yarrow (*Achillea milefolium*) extract, peppermint extract, sage extract, mallow (*Malva sylvestris*) extract, *Cnidium* rhizome extract, *Swertia japonica* extract, soy extract, Zizyphi fruit extract, wild thyme extract, tea extract, clove extract, cogongrass (*Imperata cylindrica*) extract, *Citrus* unshiu peel extract, Japanese angelica extract, calendula extract, peach seed extract, bitter orange peel extract, *Houttuynia cordata* extract, tomato extract, natto extract, ginseng extract, garlic extract, wild rose extract, *Hibiscus* extract, *Ophiopogonis* tuber extract, parsley extract, honey, witch hazel extract, *Parietalia* extract, isodonis herba, bisabolol, loquat extract, coltsfoot (*Tussilago farfara*) extract, *Petasites japonicus* extract, hoelen (*Poria* cocos) extract, butcherbroom (*Ruscus aculeatus*) extract, grape extract, propolis, sponge gourd (*Luffa cylindrica*) extract, safflower extract, peppermint extract, linden extract, *Paeonia suffruticosa* root extract, hops extract, *Pinus sylvestris* cone extract, horse chestnut extract, white arum (*Lysichitum camtschatcense*) extract, mukurossi (*Sapindus mukurossi*) peel extract, lemon balm (*Melissa officinalis*) extract, peach extract, cornflower (*Centaurea cyanus*) extract, eucalyptus extract, saxifrage extract, *Citrus junos* extract, coix seed extract, Japanese mugwort (*Artemisia princeps*) extract, lavender extract, apple extract, lettuce extract, lemon extract, milk-vetch (*Astraglus sinicus*) extract, rose extract, rosemary extract, Roman chamomile (*Anthemis nobilis*) extract, and royal jelly extract.

In addition, biopolymers such as deoxyribonucleic acids, mucopolysaccharides, sodium hyaluronate, sodium chondroitin sulfate, collagen, elastin, chitin, chitosan and hydrolyzed egg-shell membrane, amino acid derivatives such as amino acids, sarcosine and N-methyl-L-serine, moisturizing components such as sodium lactate, urea, sodium pyrrolidonecarboxylate, betaine, hoey and raffinose, oily components such as sphingolipid, ceramide, cholesterol, cholesterol derivatives and phospholipids, antiinflammatory agents such as ε-aminocaproic acid, glycyrrhizinic acid, β-glycyrrhetinic acid, lysozyme chloride, guaiazulene and hydrocortisone, vitamins such as vitamin A, B2, B6, C, D, E, calcium pantothenate, biotin, nicotinic acid amide and vitamin C ester, active ingredients such as allantoin, diisopropylamine dichloroacetate, 4-aminomethylcyclobexanecarboxylic acid, antioxidants such as tocopherol, carotenoid, flavonoid, tannin, lignan and saponin, cell activators such as α-hydroxy acid and β-hydroxy acid, blood circulation accelerators such as γ-orizanol and vitamin E derivatives, wound healing agents such as retinol and retinol derivatives, whitening agents such as arbutin, kojic acid, placenta extract, sulfur, ellagic acid, linoleic acid, tranexamic acid and glutathione, cepharanthine, licorice extract, red pepper tincture, hinokitiol, iodinated garlic extract, pyridoxine hydrochloride, dl-α-tocopherol, dl- α -tocopherol acetate, nicotinic acid, nicotinic acid derivatives, calcium pantothenate, D-pantothenyl alcohol, acetylpantothenyl ethyl ether, biotin, allantoin, isopropyl methylphenol, estradiol, ethynylestradiol, capronium chloride, benzalkonium chloride, diphenhydramine hydrochloride, takanal, camphor, salicylic acid, vanillylamide nonylate, vanillylamide nonanoate, piroctone olamine, glyceryl pentadecanoate, I-menthol, mononitroguaiacol, resorcin, γ-aminobutyric acid, and the like are included in the active component.

Although the blending ratio of these physiologically active components in cosmetics depends upon concentration at which the effects of the active component are expressed, generally it is preferably 0.05 to 20% by mass, and more preferably 0.1 to 15% by mass, with respect to the total amount of cosmetic. Furthermore, it is preferable to blend one type or two or more types in combination of physiologically active component.

In addition, examples of moisturizers include ethylene glycol, propylene glycol, butylene glycol, diethylene glycol, triethylene glycol, dipropylene glycol, glycerin, diglycerin, sorbitol, maltitol, trehalose, raffinose, xylitol, mannitol, hyaluronic acid and its salts, polyglycerine and other glycols, polyvalent alcohols and polysaccharides.

Although there are no particular restrictions on the cosmetics of the present invention, preferable examples include skin care products; hair products, antiperspirants, makeup products and ultraviolet protective products. Although examples of these include basic cosmetics such as milky lotion, cream, lotion, sun screen, sun tan agent, anti-acne cosmetics, essence and cosmetic base, makeup cosmetics such as, foundation, whitening powder, eye shadow, concealer, eye liner, eye brow liner, mascara, cheek and face powder, nail polish, lipstick, lip liner, and lip gloss, and rinse, conditioner, hair color, setting agent, hair restorer, deodorant and perfume, in particular, oily solid cosmetics such as lipstick, concealer and eye liner, and powder foundation, powder eye shadow, face powder, powdered cheek color and other powdered solid cosmetics, as other finishing cosmetics or cosmetic bases and agents serving as cosmetic base and sun screen and so forth can remarkably exhibit the effects of the present invention. In addition, there are no particular restrictions on product form; the present invention can be applied to liquids, milky lotions, creams, solids, pastes, gels, powders, multilayered forms, mousses, sprays and sticks. Although there are no particular restrictions on the cosmetics of the present invention, preferable examples include skin care products, hair products, antiperspirants, makeup products and ultraviolet protective products. Although examples of these include basic cosmetics such as milky lotion, cream, lotion, sun screen, sun tan agent, anti-acne cosmetics, essence and cosmetic base, makeup cosmetics such as, foundation, whitening powder, eye shadow, concealer, eye liner, eye brow liner, mascara, cheek and face powder, nail polish, lipstick, lip liner, and lip gloss, and rinse, conditioner, hair color, setting agent, hair restorer, deodorant and perfume, in particular, oily solid cosmetics such as lipstick, concealer and eye liner, and powder foundation, powder eye shadow, face powder, powdered cheek color and other powdered solid cosmetics, as other finishing cosmetics or cosmetic bases and agents serving as cosmetic base and sun screen and so forth can remarkably exhibit the effects of the present invention. In addition, there are no particular restrictions on product form; the present invention can be applied to liquids, milky lotions, creams, solids, pastes, gels, powders, multilayered forms, mousses, sprays and sticks.

Although the benefits of various optical effects can be enjoyed even if the cosmetics of the present invention are used as is, even more effective makeup can be obtained by various cosmetic methods. For example, in the case of using a cosmetic selected from foundation, eye shadow or lipstick and so forth after using a cosmetic base blended with a powder obtained by crushing an inorganic substance having crystallinity and transparency, the coloring of the foundation, eye shadow, lipstick or other makeup cosmetic applied thereon is improved due to the effects of the cosmetic base dispersing and reflecting light,, so that the effect of appearing more attractive can be expected. Conversely, by using a finishing cosmetic blended with a transparent inorganic powder of the present invention, such as face powder or cheek powder on a cosmetic selected from foundation, eye shadow, lipstick and so forth used in advance, for example, by using a face powder or cheek powder on a foundation, a cosmetic effect of imparting a tighter feeling can be obtained. Naturally, use of these in combination can also give preferable effects.

The powder obtained by crushing an inorganic substance having a crystalline structure and transparency of the present invention has a cosmetic appearance like that of porcelain when used. While the cosmetics of the present invention are freely compatible with uses by young persons to the elderly, when considering the case of foundation only, they are capable of effectively imparting natural sense of skin luster and brightness to elderly women whose skin has diminished ability to scatter light.

### Examples

Hereinafter, the present invention will be described in detail by examples and comparative examples. Methods for evaluating the various characteristics of cosmetics used in the examples and comparative examples are indicated below.

### [Evaluation of Skin Usefulness]

Ten expert panelists were assigned to each evaluation parameter (although some panelists were assigned to more than one parameter). Evaluations were made in accordance with the evaluation standards shown in Table 1, and the total scores of all panelists were used as evaluation results. Thus, a higher score indicates a higher degree of usefulness with respect to the evaluation parameter (maximum score: 50 points). Evaluations of lipstick consisted of reduced conspicuousness of lip wrinkles, attractive appearance of lipstick color and feel (quality of feel when the lipstick was applied). Other parameters consisted of covering effects of fine wrinkles and skin roughness, obscuring effects of skin spots and skin darkness, presence of texture that resembled the skin, feel (quality of feel when applied), and whether or not there was a low degree of color darkening (evaluated for foundation only under a daylight type of fluorescent lamp). In addition, makeup methods were evaluated with respect to whether or not coloring appeared attractive and whether or not makeup appeared attractive.

**[Table 1]**

| Standard | Score |
|---|---|
| Felt to be highly effective | 5 |
| Felt to be effective | 4 |
| Felt to be somewhat effective | 3 |
| Felt to only be slightly effective | 2 |
| Not felt to be effective | 1 |

### [Evaluation of White Appearance]

The presence of a white appearance was confirmed when skin to which a sample (foundation) was applied was photographed with a camera.

### Production Example 1 (Production of Amethyst Powder)

Amethyst (purple crystal) having a diameter of 5 millimeters to 1 centimeter and a transparent appearance was coarsely crushed using a hammer, and then pulverized using a jet mill. Further, grains of 25 micrometers or larger were removed by classifying with an ultrasonic sieve to obtain an irregularly shaped powder having an average primary grain size of 13 micrometers (and a grain size distribution such that the number of grains having a grain size of 1 micrometer or less accounted for 20% by mass of the powder). After coating this powder with methyl hydrogen polysiloxane in hexane at a powder mass ratio of 3% by mass and removing the solvent, the powder was heat-treated for 5 hours at 130°C to obtain a silicone-treated amethyst powder.

### Production Example 2 (Production of Amethyst Powder)

Amethyst (purple crystal) having a diameter of 5 millimeters to 1 centimeter and a transparent appearance was coarsely crushed using a hammer and then pulverized using a jet mill. Further, grains of 25 micrometers or larger were removed by classifying with a mesh sieve to obtain an irregularly shaped powder having an average primary grain size of 13 micrometers (and a grain size distribution such that the number of grains having a grain size of 1 micrometer or less accounted for 20% by mass of the powder). This powder was then subjected to coating treatment at 5% by mass using a solution of Nε-lauroyl-L-lysine (Amihope LL, manufactured by Ajinomoto) in alkali to obtain an Amihope-treated amethyst powder.

### Production Example 3 (Production of Quartz powder)

Synthetic quartz powder for use as an optic fiber raw material having a diameter of 0.1 millimeter to 1 millimeter and a transparent appearance was pulverized using a jet mill and contaminating grains of 25 micrometers or larger were removed by classifying with an ultrasonic sieve to obtain an irregularly shaped powder having an average primary grain size of 10 micrometers (and a grain size distribution such that the number of grains having a grain size of 1 micrometer or less accounted for 20% by mass of the powder). After forming a hot water slurry of this powder with agar at 5% by mass in a mass ratio based on the powder, the slurry was dried using a spray dryer to obtain an agar-treated quartz powder.

### Production Example 4 (Production of Quartz Powder)

Quartz powder for use as an optic fiber raw material having a diameter of 0.1 millimeter to 1 millimeter and a transparent appearance was pulverized using a jet mill and contaminating grains of 25 micrometers or larger were removed by classifying with an ultrasonic sieve to obtain an irregularly shaped powder having an average primary grain size of 6 micrometers (and a grain size distribution such that the number of grains having a grain size of 1 micrometer or less accounted for 20% by mass of the powder). This powder was then subjected to coating treatment with octyltriethoxysilane at 6% by mass in a mass ratio based on the powder in toluene, followed by removal of the solvent and heat-treatment for 5 hours at 130°C to obtain an octylsilylated quartz powder.

### Production Example 5 (Production of Sapphire Powder)

Coarsely crushed synthetic sapphire crystals synthesized to a length of 9 centimeters using the Verneuil's method were pulverized in a vibrating ball mill, and contaminating grains of 25 micrometers or larger were removed by classifying with an ultrasonic sieve to obtain an irregularly shaped powder having an average primary grain size of 7 micrometers (and a grain size distribution such that the number of grains having a grain size of 1 micrometer or less accounted for 20% by mass of the powder). This powder was then subjected to coating treatment at 10% by mass using a solution of Nε-lauroyl-L-lysine (Amihope LL, Ajinomoto) in alkali to obtain an Amihope-treated sapphire powder.

### Production Example 6 (Production of Ruby Powder)

Coarsely crushed synthetic ruby crystals synthesized to a length of 6 centimeters using the Verneuil's method were pulverized by placing in a vibrating ball mill, and contaminating grains of 25 micrometers or larger were removed by classifying with an ultrasonic sieve to obtain an irregularly shaped powder having an average primary grain size of 6 micrometers (and a grain size distribution such that the number of grains having a grain size of 1 micrometer or less accounted for 20% by mass of the powder). This powder was then subjected to coating treatment at 10% by mass using a solution of Nε-lauroyl-L-lysine (Amihope LL, Ajinomoto) in alkali to obtain an Amihope-treated ruby powder.

### Production Example 7 (Production of Garnet Powder)

Natural garnet ore (crystalline) having a diameter of 5 millimeters to 15 millimeters and a transparent appearance was coarsely crushed using a hammer, and then pulverized using a jet mill. Further, contaminating grains of 25 micrometers or larger were removed by classifying with an ultrasonic sieve to obtain an irregularly shaped garnet powder having an average primary grain size of 11 micrometers (and a grain size distribution such that the number of grains having a grain size of 1 micrometer or less accounted for 20% by mass of the powder).

### Production Example 8 (Production of Silicone Elastomer Fine Powder)

50 parts by mass of silicone elastomer (Torayfil E-508, manufactured by Toray-Dow Corning Silicone) and 50 parts by mass of sericite were coarsely mixed, and the mixture was treated twice by 30 seconds of operation with a cutter mill (>11,000 rpm), while tapping to prevent the powder from becoming adhered to the inside walls of the container, to obtain a silicone elastomer fine powder. The agglomerated grain size of the pulverized product was distributed over the range of 2 to 40 micrometers as determined by observing on a scanning electron microscope.

### Production Example 9 (Silicone Elastomer Aqueous Dispersion)

Product BY29-123 manufactured by Toray-Dow Corning Silicone (50% by mass silicone elastomer water dispersion) was used.

### Production Example 10 (Production of Silicone Elastomer Kneaded Product)

After coarsely mixing 35 parts by mass of silicone elastomer (Torayfil E-508, Toray-Dow Corning Silicone) and 65 parts by mass of the volatile silicone oil, methyltrimethicone (branched tetramer methylpolysiloxane), the mixture was kneaded using a high-speed two-shaft extruder (kneader), followed by passing through a 200 mesh sieve during extrusion to obtain a paste-like silicone elastomer.

### Comparative Production Example 1

Amorphous (vitreous) quartz powder for use in optic fibers having a white appearance that is commercially available as quartz powder having an average primary grain size of 1.0 micrometer (SO-C3, manufactured by Admatechs) was subjected to coating treatment with octyltriethoxysilane at 6% by mass in a mass ratio based on the powder in toluene, followed by distilling off the solvent and heat treatment for 5 hours at 130°C to obtain octylsilylated quartz powder.

### Comparative Production Example 2

Spherical amorphous silica powder having a white appearance and an average primary grain size of 12 micrometers was coated at 3% by mass in a mass ratio based on the powder using methyl hydrogen polysiloxane in hexane, followed by removal of the solvent and heat treatment for 5 hours at 130°C to obtain silicone-treated amorphous spherical silica powder.

### Comparative Production Example 3

Amethyst (purple crystal) having a diameter of 5 millimeters to 1 centimeter and a transparent appearance was coarsely crushed using a hammer, and pulverized using a jet mill followed by using a wet ball mill to obtain an irregularly shaped powder having an average primary grain size of 1.2 micrometers. This powder was then coated at 3% in a mass ratio based on the powder using methyl hydrogen polysiloxane in hexane, followed by removal of the solvent and heat treatment at 130°C for 5 hours to obtain silicone-treated amethyst powder.

### Comparative Production Example 4

Amorphous (vitreous) quartz powder for use in optic fibers having a white appearance that is commercially available as quartz powder having an average primary grain size of 1.0 micrometer (SO-C3, manufactured by Admatechs) was used as a quartz powder in a comparative example.

### Example 1

A lipstick was produced according to the formulations shown in Table 2 and the production method below. Furthermore, Amihope-treated pigments that were surface-treated in the same manner as Production Examples 5 and 6 were used as the Amihope-treated pigments. Note that all the units are in % by mass.

**[Table 2]**

| Component | Blending Amount |
|---|---|
| (1) Paraffin wax | 6 |
| (2) Ceresin | 3 |
| (3) Microcrystalline wax | 5 |
| (4) Candelilla wax | 3 |
| (5) Diglyceryl triisostearate | Balance |
| (6) Hydrogenated polybutene (molecular weight 1.000) | 20 |
| (7) Dipentaerythritol fatty acid ester | 10 |
| (8) Vaseline | 5 |
| (9) Liquid paraffin | 5 |
| (10) Glyceryl trioctanoate | 15 |
| (11) dl-tocopherol | 0.2 |
| (12) Amihope-treated amethyst powder (Production Example 2) | 1 |
| (13) Amihope-treated sapphire powder (Production Example 5) | 1 |
| (14) Amihope-treated ruby powder (Production Example 6) | 1 |
| (15) Amihope-treated Red No. 202 | 0.4 |
| (16) Amihope-treated Yellow No. 4 aluminum lake | 0.2 |
| (17) Amihope-treated mica titanium | 3 |
| (18) Amihope-treated red iron oxide-coated titanated mica | 5 |

### Production Method:

After melting components (1) to (11) at 90°C, components (12) to (16) were mixed in, and further mixing and crushing was performed using a roller. Thereaftrer, components (17) and (18) were dispersed in the resulting mixture, followed by re-melting and degassing. The resultant was filled into a mold, removed after cooling and placed in a container to obtain a finished product.

### Comparative Example 1

A finished product was obtained in the same manner as that in Example 1 with the exception of using Amihope-treated vitreous quartz powder (Comparative Production Example 1) instead of the Amihope-treated sapphire powder (Production Example 5) and Amihope-treated ruby powder (Production Example 6) used in Example 1.

### Example 2 (Reference)

A powder foundation was produced according to the formulations shown in Table 3 and the production method below. Furthermore, the agar-treated pigment shown in the table was produced in compliance with the surface treatment method of Production Example 3. All the units in the table are in % by mass.

**[Table 3]**

| Component | Blending Amount |
|---|---|
| Agar-treated red iron oxide | 1 |
| Agar-treated yellow iron oxide | 3 |
| Agar-treated black iron oxide | 0.5 |
| Agar-treated titanium oxide | 14 |
| Agar-treated talc | balance |
| Agar-treated barium sulfate | 20 |
| Agar-treated sericite | 7.5 |
| Agar-treated quartz powder (Production Example 3) | 17 |
| Liquid paraffin | 3 |
| Propylene glycol dicaprylate | 3 |
| Isoparaffin wax | 4 |
| Dimethylpolysiloxane | 3 |
| Trifluoropropylated dimethiconol | 1 |
| Antiseptics | Adequate amount |

### Production Method:

The oily components were heated and mixed and then slowly added from above to the pre-mixed powder components, followed by stirring, passing through a 60 mesh sieve, and molding in a metal dish using a mold to obtain a finished product.

### Comparative Example 2

A finished product was obtained in the same manner as that in Example 2 with the exception of using the same agar-treated talc as that used in Example 2 instead of the agar-treated quartz powder (Production Example 3) of Example 2 (an example when a transparent inorganic powder having crystallinity was not blended).

### Example 3 (Reference)

A powder foundation was produced according to the formulations shown in Table 4 and production method below.

**[Table 4]**

| Component | Blending Amount |
|---|---|
| Silicone-treated red iron oxide | 1 |
| Silicone-treated yellow iron oxide | 3 |
| Silicone-treated black iron oxide | 0.5 |
| Silicone-treated titanium oxide | 12 |
| Silicone-treated talc | Balance |
| Silicone-treated barium sulfate | 20 |
| Silicone-treated sericite | 7.5 |
| Silicone-treated amethyst powder (Production Example 1) | 12 |
| Liquid paraffin | 3 |
| Caprylic/capric triglyceride | 5 |
| Isoparaffin wax | 4 |
| Dimethylpolysiloxane | 2 |
| Trifluoropropylated dimethiconol | 1 |
| Antiseptics | Adequate amount |

### Production Method

The oily components were heated and mixed and then slowly added from above to the pre-mixed powder components, followed by stirring, passing through a 60 mesh sieve, and molding into the shape of a metal dish using a mold to obtain a finished product.

### Comparative Example 3

A finished product was obtained in the same manner as that in Example 3 with the exception of using the silicone-treated amorphous spherical silica powder produced in Comparative Production Example 2 instead of the silicone-treated amethyst powder (Production Example 1) used in Example 3 (an example of a difference between crystalline and amorphous materials).

### Comparative Example 4

A finished product was obtained in the same manner as that in Example 3 with the exception of using the silicone-treated amethyst powder having an average primary grain size of 1.2 micrometers produced in Comparative Production Example 3 instead of the silicone-treated amethyst powder (Production Example 1) used in Example 3 (an example of a difference in average grain size).

### Example 4 (Reference)

An agent serving as cosmetic base and sun screen was produced according to the formulations shown in Table 5 and the production method below. Note that 12% by mass octylsilane-treated silica alumina-treated titanium oxide (average primary grain size: 12 nanometers) was used as the octylsilylated fine grain titanium oxide, 5% methyl hydrogen polysiloxane (HRS-2, manufactured by Shin-Etsu Chemical)-treated fine grain zinc oxide (ZnO-350, manufactured by Sumitomo Osaka Cement) was used as the silicone-treated fine grain zinc oxide, and KF-6019 manufactured by Shin-Etsu Chemical was used as the polyether-modified silicone.

**[Table 5]**

| Component | Blending Amount |
|---|---|
| Octylsilylated quartz powder (Production Example 4) | 15 |
| Octylsilylated titanium oxide fine particles | 3 |
| Silicone-treated zinc oxide fine particles | 8 |
| Polyether-modified silicone | 3 |
| Dimethylpolysiloxane | 3 |
| High polymerization degree dimethylpolysiloxane | 0.3 |
| Trifluoropropylated trimethyl siloxysilicate | 1 |
| Decamethylcyclopentasiloxane | Balance |
| Methyl trimethicone | 15 |
| Dipropylene glycol | 3 |
| Raffinose | 0.5 |
| Purified water | 20 |
| Antiseptics | Adequate amount |

### Production Method

After mixing the pigment and oily agents and crushing using a bead mill, the hydrophilic components were mixed in. The mixture was then filled into a plastic bottle along with stainless steel balls to obtain a finish product.

### Comparative Example 5

A finished product was obtained in the same manner as that in Example 4 with the exception of using the octylsilylated quartz powder of Comparative Production Example 1 instead of the octylsilylated quartz powder (Production Example 4) used in Example 4 (an example of a difference between crystalline and amorphous states of the same material).

### Example 5

A face powder was produced according to the formulations shown in Table 6 and production method. Note that the same surface-treated Amihope-treated pigment as those in Production Examples 5 and 6 was used as the Amihope-treated pigment. In addition, all the units are in % by mass.

**[Table 6]**

| | |
|---|---|
| Amihope-treated sapphire powder (Production Example 5) | 5 |
| Amihope-treated ruby powder (Production Example 6) | 20 |
| Amihope-treated talc | Balance |
| Amihope-treated mica | 40 |
| Amihope-treated sericite | 10 |
| Amihope-treated low sheen titanated mica | 1 |
| Amihope-treated titanated mica | 3 |
| Isononyl isononanoate | 2 |
| Antiseptics | Adequate amount |

### Production Method

Each of the components was mixed and the resultant was filled into a container to obtain a finished product.

### Example 6 (Reference)

After using the agent serving as cosmetic base and sun screen agent produced in Example 4, a commercially available two-way powder type foundation was used.

### Comparative Example 6

After using a commercially available lotion not blended with a pigment, the same commercially available two-way powder type foundation as that used in Example 6 was used.

### Example 7

A commercially available two-way powder type foundation was used after fixing the skin using a lotion, and the face powder produced in Example 5 was then applied over the foundation.

### Comparative Example 7

In accordance with Example 7, after fixing the skin using a lotion, a commercially available two-way powder type foundation was used and then a face powder produced using an Amihope-treated talc was applied over the foundation instead of Amihope-treated sapphire powder (Production Example 5) and Amihope-treated ruby powder (Production Example 6).

### Example 8 (Reference)

The foundation of Example 3 was used after using the cosmetic base of Example 4.

The results of evaluating the examples and comparative examples are shown in Tables 7 to 9.

**[Table 7]**

| | Reduced conspicuousness of lip wrinkles | Active appearance of lipstick color | Feel (quality of feel when applied) |
|---|---|---|---|
| Example 1 | 46 | 43 | 44 |
| Comparative Example 1 | 29 | 27 | 24 |

**[Table 8]**

| | Covering effect | Obscuring effect | Presence of texture | Feel | Whitening | Color darkening |
|---|---|---|---|---|---|---|
| Example 2 (Reference) | 44 | 43 | 43 | 44 | None | Low |
| Comparative Example 2 (Reference) | 22 | 15 | 30 | 40 | Some | Some |
| Example 3 (Reference) | 41 | 42 | 44 | 46 | None | Low |
| Comparative Example 3 | 32 | 37 | 36 | 13 | Some | Some |
| Comparative Example41 | 35 | 37 | 40 | 43 | Some | Low |
| Example | 41 | 41 | - | 41 | - | - |
| Comparative xample 5 | 19 | 13 | - | 25 | - | - |

**[Table 9]**

| | Feel attractive coloring | Attractive makeup |
|---|---|---|
| Example 6 (Reference) | 41 | 42 |
| Comparative Example 6 | 33 | 35 |
| Example 7 | - | 45 |
| Comparative Example 7 | - | 39 |
| Example 8 (Reference) | 44 | 43 |

From the results shown in Table 7, it can be understood that the example of the present invention reduces conspicuousness of lip wrinkles, results in more attractive lipstick color (superior coloring) and has a satisfactory feel as compared with the comparative example. In contrast, wrinkles were recognized and the lipstick color lacked brightness in the case of the comparative example.

From the results shown in Table 8, it can be understood that the examples of the present invention reduce conspicuousness of fine wrinkles and skin roughness, make skin spots and skin darkness less conspicuous while still appearing natural, result in makeup having a texture that resemble that of skin, regulate whitening during photography, have a superior feel and demonstrate inconspicuous color darkening.

In contrast, Comparative Example 2 is a comparative example when an inorganic substance having a crystalline structure and transparency was not blended, and it can be seen that the intended object of the present invention cannot be exhibited.

Comparative Example 3 is an example of differences attributable to crystalline and amorphous properties of the material, and considerable differences in the resulting optical characteristics were observed depending on the presence or absence of crystalline properties even when the same silicon oxide type compound was used.

Comparative Example 4 confirmed differences attributable to average primary grain size in the same amethyst material, and optical characteristics were determined to be inferior overall to those of Example 3 when the average primary grain size became 1.2 micrometers.

Comparative Example 5 confirmed differences attributable to crystalline and amorphous properties in the same quartz material, and optical characteristics were determined to change considerably only in the case of amorphous properties.

From the results shown in Table 9, it can be understood that in the case the makeup method of the present invention is used for a cosmetic base, the method exhibited effects of improving coloring and attractive appearance of makeup. In addition, when used for finishing cosmetics, the method of the present invention exhibited the effect that makeup appeared more attractive. Further, when used for a combination of cosmetic base and foundation, the method of the present invention improved coloring and appearance more effectively. From these, it can be seen that optical effects are maintained even when the cosmetic base and foundation are used in combination.

### Example 9 (Reference)

A powder foundation was produced according to the formulations shown in Table 10 and the production method below. Note that a silicone-treated pigment produced using the same treatment method as that in Production Example 1 was used for the silicone-treated pigment. All the units of blending amounts are % by mass.

**[Table 10]**

| Component | Blending Amount |
|---|---|
| [Step 1] | |
| (Component A) | |
| Silicone elastomer water dispersion (Production Example 9) | 60 |
| Plate-like barium sulfate | 10 |
| Garnet powder (Production Example 7) | 30 |
| Talc | 30 |
| (Component B) | |
| Purified water | 15 |
| (Component C) | |
| Trifluoropropylated dimethiconol | 2 |
| [Step 2] | |
| Composite powder of Step 1 | 60 |
| Silicone-treated red iron oxide | 1 |
| Silicone-treated yellow iron oxide | 3 |
| Silicone-treated black iron oxide | 0.5 |
| Silicone-treated titanium oxide | 12 |
| Silicone-treated sericite | Balance |
| Liquid paraffin | 2 |
| Caprylic/capric triglyceride | 4 |
| Isoparaffin wax | 3 |
| Dimethylpolysiloxane | 2 |
| Antiseptics | Adequate amount |

### Production Method

After uniformly mixing component A and component B of step 1, component C was added and further mixed. Next, the moisture was removed by heating in a fluidized bed dryer, followed by granulation to obtain a compound powder coated with trifluoropropylated dimethiconol. Next, the oily components of step 2 were heated and mixed and then slowly added from above to the pre-mixed powder components, followed by stirring, passing through a 60 mesh sieve, and molding into the shape of a metal dish using a mold to obtain a finished product.

### Comparative Example 8

A finished product was obtained in the same manner as that in Example 9 with the exception of using the amorphous quartz powder of Comparative Production Example 4 instead of the garnet powder (Production Example 7) used in Example 9 (example of differences attributable to crystalline and amorphous properties of the material used).

### Example 10 (Reference)

A powder foundation was produced according to the formulation shown in Table 11 and the production method below.

**[Table 11]**

| Component | Blending Amount |
|---|---|
| Silicone-treated red iron oxide | 1 |
| Silicone-treated yellow iron oxide | 3 |
| Silicone-treated black iron oxide | 0.5 |
| Silicone-treated titanium oxide | 13 |
| Silicone-treated talc | Balance |
| Silicone-treated sericite | 25 |
| Silicone-treated amethyst powder (Production Example 1) | 15 |
| Silicone elastomer pulverized powder | |
| (Production Example 8) | 5 |
| Liquid paraffin | 3 |
| Caprylic/capric triglyceride | 4 |
| Isoparaffin wax | 3 |
| Dimethylpolysiloxane | 4 |
| Antiseptics | Adequate amount |

### Production Method:

The oily components were heated and mixed and then slowly added from above to the pre-mixed powder components, followed by stirring, passing through a 60 mesh sieve, and molding into the shape of a metal dish using a mold to obtain a finished product.

### Example 11 (Reference)

A finished product was obtained in the same manner as that in Example 10 with the exception of using silicone-treated talc instead of the silicone elastomer pulverized powder (Production Example 8) used in Example 10 (example of using only a transparent inorganic powder having crystallinity without using a silicone elastomer).

### Comparative Example 9

A finished product was obtained in the same manner as that in Example 10 with the exception of using silicone-treated talc instead of the silicone-treated amethyst powder (Production Example 1) used in Example 10 (example of using only a silicone elastomer without using a transparent inorganic powder having crystallinity).

### Comparative Example 10

A finished product was obtained in the same manner as that in Example 10 with the exception of using a silicone-treated talc instead of the silicone elastomer pulverized powder (Production Example 8) and the silicone-treated amethyst powder (Production Example 1) used in Example 10 (example when neither a transparent inorganic powder having crystallinity nor a silicone elastomer was used).

### Example 12 (Reference)

A cosmetic base was produced according to the formulation shown in Table 12 and the production method below.

**[Table 12]**

| Component | Blending Amount |
|---|---|
| Silicone Elastomer Kneaded Product | |
| (Production Example 10) | Balance |
| Trifluoropropylated dimethiconol gum | |
| (50% Decamethylcyclopentasiloxane solution) | 8 |
| Garnet powder (Production Example 7) | 7 |
| Antiseptics | Adequate amount |

### Production Method:

After mixing each component, the mixture was filled into a tube container to obtain a finished product.

### Comparative Example 11

A finished product was obtained in the same manner as that in Example 12 with the exception of using spherical amorphous silica powder having an average primary grain size of 11 micrometers instead of the garnet powder (Production Example 7) used in Example 12.

The results of evaluating the examples and comparative examples are shown in Table 13.

**[Table 13]**

| | Reduced conspicuousness of fine wrinkles and skin roughness | Satisfactory feel during application |
|---|---|---|
| Example 9 (Reference) | 46 | 45 |
| Comparative Example 8 | 38 | 35 |
| Example 10 (Reference) | 48 | 48 |
| Example 11 (Reference) | 40 | 43 |
| Example 9 (Reference) | 36 | 35 |
| Comparative Example 10 | 20 | 27 |
| Example 12 (Reference) | 50 | 41 |
| Comparative Example 11 | 44 | 16 |

From the results shown in Table 13, it can be seen that the examples of the present diclosure serve to reduce the conspicuousness of fine wrinkles and skin roughness and have a superior feel (feel during application) compared with the comparative examples. In addition, in the case of applying foundation over the cosmetic base of Example 12, the effect was observed that enabled the skin color of the foundation to appear bright. This effect was observed more prominently in the case of using the foundation of Example 10 over the cosmetic base of Example 12. In contrast, although Comparative Example 8 confirmed differences attributable to crystalline properties and amorphous properties with the same quartz material, since covering effects decrease simply as a result of having amorphous properties, it can be seen that inorganic substances having a crystalline structure and transparency were determined to exhibit their effects synergistically with the silicone elastomer.

In addition, Example 10 is an example of using only an inorganic powder having a crystalline structure and transparency without using a silicone elastomer, Comparative Example 9 is an example of using only a silicone elastomer without using an inorganic powder having a crystalline structure and transparency, and Comparative Example 10 is an example of not using an inorganic powder having a crystalline structure and transparency or a silicone elastomer. From the results of these experiments as well, it can be seen that inorganic powders having a crystalline structure and transparency demonstrate their effects synergistically with the silicone elastomer. Comparative Example 11 indicates the case of using a known spherical amorphous silica powder that is frequently used in cosmetics, and it can be seen that Comparative example 11 has weak optical effects as compared with Example 12 of the present invention. In addition, when color darkening over time during testing of foundation was assessed visually, it can be seen that the cosmetics of the present invention has characteristics that they cause color darkening to some extent but the color darkening is inconspicuous.

### INDUSTRIAL APPLICABILITY

As has been described above, the present invention has superior fine wrinkle and skin darkening covering effects as well as skin spot and skin darkness obscuring effects, exhibits little color darkening over time and provides a cosmetic coating film having a texture close to skin in optical deepness by blending an inorganic powder having a crystalline structure and transparency.

In addition, cosmetics are obtained that can effectively reduce the conspicuousness of fine wrinkles and skin roughness, have a superior feel when applied and reduce the conspicuousness of color darkening by blending a silicone elastomer into an inorganic powder having a crystalline structure and transparency.

The present invention also provides cosmetics that regulate whitening during photography and have a superior feel and coloring, as well as a makeup method that has superior effects in improving makeup effects such as improving the coloring of foundation and lipstick.

The present invention can be used in skin care products, hair products, antiperspirants, makeup products, ultraviolet protective products and so forth, and is applied particularly preferably to oily solid cosmetics such as lipstick, concealer and eye liner, powdered solid cosmetics such as powder foundation, powder eye shadow, face powder and powdered cheek color, as finishing cosmetics or cosmetic bases and combined cosmetic bases, and agents serving as sun screens.

## Claims

1. A cosmetic comprising crushed inorganic powder of sapphire having a crystalline structure and transparency;
wherein the number of grains of the inorganic powder having a primary grain size of 25 micrometers or more is less than 15 %,
and wherein the average primary grain size of the inorganic powder is in a grain size distribution range of 5 to 15 micrometers,
and the cosmetic further containing a spherical silicone elastomer powder having a primary grain size in the range of 1 to 50 micrometers.

2. The cosmetic according to claim 1, wherein the major axis of said inorganic substance before crushing is in the range of 0.1 millimeter to 100 centimeters.

3. The cosmetics according to claim 1 or 2, wherein said crushing is performed in a ball mill or jet mill, and followed by classification.

4. The cosmetic according to any one of claims 1 to 3, wherein said inorganic powder is further subjected to coating treatment with a hydrophilic or hydrophobic surface treatment agent, selected from N-acylated lysine, alkylsilane and silicone.

5. The cosmetic according to any one of claims 1 to 4, wherein said silicone elastomer is blended in one or more kinds of states selected from being pulverized, being mixed by crushing or kneading with an oily agent, and being dispersed in an aqueous system.

6. The cosmetic according to any one of claims 1 to 5, wherein the blending amount of said inorganic powder in the cosmetics is in the range of 0.1 to 50% by mass.

7. The cosmetic according to any one of claims 1 to 6, wherein the blending amount of said inorganic powder in the cosmetics is in the range of 0.1 to 10% by mass.

8. The cosmetic according to any one of claims 1 to 7, wherein the cosmetic is a solid powder cosmetic.

9. The cosmetic according to any one of claims 1 to 8, wherein the cosmetic is an oily solid cosmetic.

10. A makeup method comprising applying the cosmetic according to any one of claims 1 to 9 onto the skin as a foundation base and applying another cosmetic selected from foundation, eye shadow and lipstick onto the foundation base.

11. A makeup method comprising applying a cosmetic selected from foundation, eye shadow and lipstick onto a skin and then applying the cosmetic according to any one of claims 1 to 9 as a finishing cosmetic onto the cosmetic.

12. A make up method comprising applying to any one of claims 1 to 9 onto the skin to cover fine wrinkles and skin roughness, obscure skin spots and darkness, and regulate whitening during photography.

13. Process for the preparation of a cosmetic according to any one of claims 1 to 12 comprising the following steps:
a) Selecting sapphire having a crystalline structure and transparency, wherein the major axis of said sapphire is in the range of 0.1 millimeter to 100 centimeters;
b) Crushing said sapphire so that the number of grains having a primary grain size of 25 micrometers or more is less than 15 %, and the average primary grain size is in a grain size distribution range of 5 to 15 micrometers; and
c) Blending the crushed sapphire at 0.1 to 98 % by mass in a cosmetic.

14. Process according to claim 13, wherein the inorganic material selected has a primary grain size of at least 100 micrometers before crushing.

15. Process according to claims 13 or 14, wherein the inorganic material selected has a refractive index of 1.45 to 3.0, preferably from 1.5 to 2.95, before crushing.

## Patentansprüche

1. Kosmetikprodukt umfassend zerstoßenes anorganisches Pulver von Saphir mit einer kristallinen Struktur und Transparenz;
wobei die Anzahl von Körnchen des anorganischen Pulvers mit einer primären Korngröße von 25 µm oder mehr geringer ist als 15 %;
und wobei die durchschnittliche primäre Korngröße des anorganischen Pulvers in einem Korngrößenverteilungsbereich von 5 bis 15 µm liegt,
und das Kosmetikprodukt weiterhin ein kugelförmiges Silikonelastomerpulver mit einer primären Korngröße im Bereich von 1 bis 50 µm enthält.

2. Kosmetikprodukt nach Anspruch 1, wobei die Hauptachse der anorganischen Substanz vor dem Zerstoßen im Bereich von 0,1 mm bis 100 cm liegt.

3. Kosmetikprodukt nach Anspruch 1 oder 2, wobei das Zerstoßen in einer Kugelmühle oder Strahlmühle durchgeführt wird, worauf Klassifizieren folgt.

4. Kosmetikprodukt nach einem der Ansprüche 1 bis 3, wobei das anorganische Pulver weiterhin einer Beschichtungsbehandlung mit einem hydrophilen oder hydrophoben Oberflächenbehandlungsmittel, ausgewählt aus N-acetyliertem Lysin, Alkylsilan und Silikon, unterzogen wird.

5. Kosmetikprodukt nach einem der Ansprüche 1 bis 4, wobei das Silikonelastomer in einer oder mehreren Zuständen gemischt wird, ausgewählt daraus, dass es pulverisiert wird, dass es durch Zerstoßen oder Verkneten mit einem öligen Mittel gemischt wird und dass es in einem wässrigen System dispergiert wird.

6. Kosmetikprodukt nach einem der Ansprüche 1 bis 5, wobei die Mischmenge des anorganischen Pulvers in der Kosmetikprodukt im Bereich von 0,1 bis 50 Masse-% liegt.

7. Kosmetikprodukt nach einem der Ansprüche 1 bis 6, wobei die Mischmenge des anorganischen Pulvers in der Kosmetikprodukt im Bereich von 0,1 bis 10 Masse-% liegt.

8. Kosmetikprodukt nach einem der Ansprüche 1 bis 7, wobei das Kosmetikprodukt ein festes Pulverkosmetikprodukt ist.

9. Kosmetikprodukt nach einem der Ansprüche 1 bis 8, wobei das Kosmetikprodukt ein öliges festes Kosmetikprodukt ist.

10. Schminkmethode umfassend das Auftragen des Kosmetikprodukts Ansprüche 1 bis 9 auf die Haut als Makeup-Grundlage und Auftragen eines weiteren Kosmetikprodukts, ausgewählt aus Grundierung, Lidschatten und Lippenstift, auf die Makeup-Grundlage.

11. Schminkmethode umfassend das Auftragen eines Kosmetikprodukts ausgewählt aus Grundierung, Lidschatten und Lippenstift, auf die Haut und dann Auftragen des Kosmetikprodukts nach einem der Ansprüche 1 bis 9 als Abschluss-Kosmetikprodukt auf das Kosmetikprodukt.

12. Schminkmethode umfassend das Auftragen nach einem der Ansprüche 1 bis 9 auf die Haut zum Abdecken von feinen Fältchen und Haut-Rauhigkeit, unbestimmten Hautflecken und Dunkelheit, und um die Hautaufhellung während des Fotografierens zu regulieren.

13. Verfahren zur Herstellung eines Kosmetikprodukts nach einem der Ansprüche 1 bis 12 umfassend die folgenden Schritte:
a. Auswählen von Saphir mit einer Kristallstruktur und Transparenz, wobei die Hauptachse des Saphirs im Bereich von 0,1 mm bis 100 cm liegt;
b. Zerstoßen des Saphirs, so dass die Anzahl der Körnchen mit einer primären Korngröße von 25 µm oder mehr weniger als 15 % beträgt, und die durchschnittliche primäre Korngröße in einem Korngrößenverteilungsbereich von 5 bis 15 µm liegt; und
c. Mischen des zerstoßenen Saphirs bei 0,1 bis 98 Masse-% in einem Kosmetikprodukt.

14. Verfahren nach Anspruch 13, wobei das ausgewählte anorganische Material eine primäre Korngröße von mindestens 100 µm vor dem Zerstoßen aufweist.

15. Verfahren nach den Ansprüchen 13 oder 14, wobei das ausgewählte anorganische Material einen Brechungsindex von 1,45 bis 3,0, vorzugsweise von 1,5 bis 2,95 vor dem Zerstoßen aufweist.

## Revendications

1. Cosmétique comprenant une poudre minérale broyée de saphir présentant une structure cristalline et une transparence ;
dans lequel le nombre de grains de la poudre minérale présentant une granulométrie primaire de 25 micromètres ou plus est inférieur à 15 %,
et dans lequel la granulométrie primaire moyenne de la poudre minérale est dans une gamme de distribution granulométrique de 5 à 15 micromètres,
et le cosmétique contient en outre une poudre de silicone élastomère sphérique ayant une granulométrie primaire dans la gamme de 1 à 50 micromètres.

2. Cosmétique selon la revendication 1, dans lequel l'axe majeur de ladite substance minérale avant broyage est dans la gamme de 0,1 millimètre à 100 centimètres.

3. Cosmétiques selon la revendication 1 ou 2, dans lesquels ledit broyage est réalisé à l'aide d'un broyeur à billes ou d'un désintégrateur à jet d'air, suivi d'une classification.

4. Cosmétique selon l'une quelconque des revendications 1 à 3, dans lequel ladite poudre minérale est ensuite soumise à un traitement d'enrobage par un agent de traitement de surface hydrophile ou hydrophobe, choisi parmi la lysine N-acylée, l'alkylsilane et le silicone.

5. Cosmétique selon l'une quelconque des revendications 1 à 4, dans lequel ledit silicone élastomère est mélangé dans un ou plusieurs types d'états choisis parmi l'état pulvérisé, l'état mélangé par broyage ou corroyage avec un agent huileux, et l'état dispersé dans un système aqueux.

6. Cosmétique selon l'une quelconque des revendications 1 à 5, dans lequel la quantité mélangée de ladite poudre minérale dans les cosmétiques est dans la gamme de 0,1 à 50 % en masse.

7. Cosmétique selon l'une quelconque des revendications 1 à 6, dans lequel la quantité mélangée de ladite poudre minérale dans les cosmétiques est dans la gamme de 0,1 à 10 % en masse.

8. Cosmétique selon l'une quelconque des revendications 1 à 7, dans lequel le cosmétique est un cosmétique en poudre solide.

9. Cosmétique selon l'une quelconque des revendications 1 à 8, dans lequel le cosmétique est un cosmétique solide huileux.

10. Méthode de maquillage comprenant l'application du cosmétique selon l'une quelconque des revendications 1 à 9 sur la peau comme base de fond de teint et l'application d'un autre cosmétique choisi parmi un fond de teint, un fard à paupière et un rouge à lèvre sur la base de fond de teint.

11. Méthode de maquillage comprenant l'application d'un cosmétique choisi parmi un fond de teint, un fard à paupière et un rouge à lèvre sur une peau, puis l'application du cosmétique selon l'une quelconque des revendications 1 à 9 comme cosmétique de finition sur le cosmétique.

12. Méthode de maquillage comprenant l'application selon l'une quelconque des revendications 1 à 9 sur la peau pour couvrir de fines rides et une rugosité de la peau, masquer les taches et zones sombres sur la peau, et contrôler le blanchiment durant la photographie.

13. Procédé de préparation d'un cosmétique selon l'une quelconque des revendications 1 à 12 comprenant les étapes suivantes :
a) choix du saphir présentant une structure cristalline et une transparence, dans lequel l'axe majeur dudit saphir est dans la gamme de 0,1 millimètre à 100 centimètres ;
b) broyage dudit saphir de telle façon que le nombre de grains ayant une granulométrie primaire de 25 micromètres ou plus soit inférieur à 15 %, et la granulométrie primaire moyenne soit dans une gamme de distribution granulométrique de 5 à 15 micromètres ; et
c) mélange du saphir broyé à raison de 0,1 à 98 % en masse dans un cosmétique.

14. Procédé selon la revendication 13, dans lequel le matériau minéral choisi a une granulométrie primaire d'au moins 100 micromètres avant broyage.

15. Procédé selon les revendications 13 ou 14, dans lequel le matériau minéral choisi a un indice de réfraction de 1,45 à 3,0, de préférence de 1,5 à 2,95, avant broyage.
